# EUROPÄISCHE PATENTSCHRIFT

(11) **EP 4 412 672 B1**
(45) Veröffentlichungstag und Bekanntmachung des Hinweises auf die Patenterteilung: **09.12.2026**
(21) Anmeldenummer: 22799883.8
(22) Anmeldetag: 29.09.2022
(51) Int. Cl.: A61L 31/02, A61L 31/06, A61L 31/08, A61L 31/14, A61L 31/16

(54) **BIOKORRODIERBARER FESTKÖRPER UND VERFAHREN ZUR BESCHICHTUNG EINES FESTKÖRPERS**
BIOCORRODIBLE SOLID BODY AND METHOD FOR COATING A SOLID BODY
CORPS SOLIDE BIOCORRODABLE ET PROCÉDÉ DE REVÊTEMENT D'UN CORPS SOLIDE

(30) Priorität: 05.10.2021 DE 102021125789
(43) Veröffentlichungstag der Anmeldung: 14.08.2024
(73) Patentinhaber: Bio-Gate AG, 90411 Nürnberg (DE)
(72) Erfinder: STEINRÜCKE, Peter, 90461 Nürnberg (DE)
(74) Vertreter: Meyer, Rudolf
(86) Internationale Anmeldenummer: PCT/EP2022/077157
(87) Internationale Veröffentlichungsnummer: WO 2023/057310

(56) Entgegenhaltungen:
- EP-B1- 1 790 224

## Beschreibung

Die Erfindung betrifft einen biokorrodierbaren Festkörper in Form eines biokorrodierbaren Implantats. Ferner betrifft die Erfindung ein Verfahren zur Beschichtung eines solchen Festkörpers, nämlich Implantats.

Ein Verfahren zur Behandlung einer Oberfläche eines metallischen Implantats aus einem biologisch abbaubaren Material ist zum Beispiel aus der WO 2012/007181 A1 bekannt. Zur Beschichtung eines Implantats wird in diesem Fall ein dispergiertes System verwendet, welches ein Apatit-Pulver enthält. Durch Anlegen einer Wechselspannung soll sich das Apatit in eine Oxidschicht umwandeln. Das Verfahren nach der WO 2012/007181 A1 soll sich insbesondere für die Beschichtung von Implantaten auf Magnesiumbasis eignen.

Unter Biokorrosion bezeichnet man allgemein die durch Aktivitäten von Lebewesen bewirkte Veränderung von Werkstoffen, die zu deren Zerstörung oder zur Beschädigung von damit verbundenen oder daraus bestehenden Gegenständen führt. Biokorrosion führt in der Regel zu einer Beschleunigung des Materialverfalls. Typische biologische Verursacher können Bakterien, Pilze, Hefe aber auch höhere Organismen wie zum Beispiel Pflanzen sein. Bakterielle Biofilme spielen bei der Biokorrosion wegen ihrer Vielfalt und Robustheit bei sehr hoher Organismendichte eine herausragende Rolle.

Aus der EP 1 790 224 B1 ist ein antimikrobielles Schichtmaterial bekannt, welches eine Biozid-Schicht und eine die Biozid-Schicht bedeckende Transportkontrollschicht umfasst. Bei einem Wirkstoff der Biozid-Schicht handelt es sich um Silber, Kupfer, Zink, deren Ionen oder deren Metallkomplexe oder eine Mischung oder Legierung aus den genannten Elementen. Die Transportkontrollschicht weist eine Dicke und Porosität auf, die eingestellt sind, um den bioziden Wirkstoff durch die Transportkontrollschicht hindurch in einer antimikrobiellen und nicht zytotoxischen Menge abzugeben. Die Transportkontrollschicht enthält Silizium, Kohlenstoff und Sauerstoff in bereichsmäßig definierten Anteilen. Das Schichtmaterial nach der EP 1 790 224 B1 kann zum Beispiel auf eine Polyurethan-Oberfläche aufgebracht werden. Als mögliche Verfahren zur Herstellung der Transportkontrollschicht sind Sputtern und Plasmapolymerisation genannt. Insgesamt ist das Schichtmaterial nach der EP 1 790 224 B1 unter anderem zur Beschichtung von Knochennägeln und anderen Knochenimplantaten geeignet. Das Schichtmaterial ist insbesondere dazu ausgelegt, korrosive Angriffe von Körperflüssigkeiten zu unterbinden.

Ein in der EP 1941918 A2 beschriebenes Verfahren zur Herstellung einer korrosionshemmenden Beschichtung auf einem Implantat, nämlich Stent, aus einer biokorrodierbaren Magnesiumlegierung umfasst das Behandeln einer Implantatsoberfläche mit einer wässrigen oder alkoholischen Konversionslösung, welche bestimmte Ionen in einer Konzentration im Bereich von 0,01 mol/I bis 2 mol/I enthält. Mit dem in der EP 1 941 918 A2 vorgeschlagenen Verfahren wird das Ziel einer nur temporären Inhibition der Korrosion verfolgt.

Ein weiteres Verfahren zur Herstellung einer korrosionshemmenden Beschichtung auf einem Implantat aus einer biokorrodierbaren Magnesiumlegierung ist in der EP 2 189 170 A1 beschrieben. In diesem Fall erfolgt eine anodische plasmachemische Behandlung einer Implantatsoberfläche in einem wässrigen, fluoridfreien Elektrolyten, der Ammoniak, Phosphorsäure und Borsäure enthält. Mit dem vorgeschlagenen Verfahren soll eine Beschichtung mit einer Dicke im Bereich von 1 µm bis 10 µm erzeugbar sein. Die Beschichtung soll für eine Beladung mit einem pharmazeutischen Wirkstoff geeignet sein.

In der EP 2 033 668 A2, welche ebenfalls ein Implantat aus einer biokorrodierbaren Magnesiumlegierung zum Gegenstand hat, wird eine Beschichtung vorgeschlagen, welche ein biokorrodierbares Polyphosphazen enthält.

Die WO 2010/017959 A2 beschreibt ein Implantat aus einer Magnesiumlegierung, bei welcher die Porosität zum Kern hin zunimmt. Als mögliche Beschichtungen sind in diesem Fall Oxidschichten sowie Polymere, zum Beispiel Poly-L-milchsäure, genannt, welche sich innerhalb einer bestimmten Verweildauer auflösen.

Eine in der EP 2 726 558 B2 beschriebene Beschichtungszusammensetzung umfasst eine Bindemittelmatrix auf Polysiloxan-Basis. Weiter umfasst diese Beschichtungszusammensetzung hydrophile Oligomer-/Polymer-Einheiten.

Der Erfindung liegt die Aufgabe zu Grunde, gegenüber dem Stand der Technik weiterentwickelte Möglichkeiten der gezielten Biokorrosion von Beschichtungen sowie damit zumindest teilweise überzogenen Gegenständen, nämlich Implantaten, anzugeben.

Diese Aufgabe wird erfindungsgemäß gelöst durch einen biokorrodierbaren Festkörper mit den Merkmalen des Anspruchs 1. Ebenso wird die Aufgabe gelöst durch ein Verfahren zum Beschichten eines Festkörpers, nämlich Implantats, gemäß Anspruch 10 Im Folgenden im Zusammenhang mit dem Beschichtungsverfahren erläuterte Ausgestaltungen und Vorteile der Erfindung gelten sinngemäß auch für den beschichteten Gegenstand, das heißt das beschichtete Implantat, sowie umgekehrt.

Der biokorrodierbare Festkörper, das heißt das Implantat, umfasst einen Grundwerkstoff und eine diesen zumindest teilweise abdeckende Beschichtung, welche Silizium enthält. Insbesondere kann es sich hierbei um eine Beschichtung der in der EP 1 790 224 B1 beschriebenen Art handeln. Die Beschichtung ist derart auf den Grundwerkstoff aufgebracht, dass sich unterschiedliche Korrosionsgeschwindigkeiten in verschiedenen Bereichen des Festkörpers einstellen, wobei Teile des Festkörpers unbeschichtet bleiben können.

Der beschichtete Festkörper kann entweder in einem Lebewesen oder in einer sonstigen Umgebung, in welcher ein Abbau des Festkörpers durch Biokorrosion auftritt, zum Einsatz kommen. In allen Fällen ist die Biodegradierbarkeit mit Hilfe der Beschichtung derart einstellbar, dass der Prozess der Biokorrosion und damit auch die Entstehung von Abbauprodukten nicht an der gesamten Oberfläche des Festkörpers mit einer einheitlichen Abbaurate voranschreitet. Mit der Verwendung von Silizium kann dies sogar gelingen, wenn die elementare Zusammensetzung der Beschichtung einheitlich ist. Insbesondere können sich dabei verschiedene Abschnitte der Beschichtung hinsichtlich ihrer Porosität voneinander unterscheiden, wobei die Porosität signifikanten Einfluss auf die hydrophilen oder hydrophoben Eigenschaften der Beschichtung hat. Ferner kann eine uneinheitliche Dicke der Beschichtung gegeben sein. Als mögliche Bestandteile der Beschichtung sind auch Kunststoffe, beispielsweise Polyethylen, zu nennen. In jedem Fall unterscheiden sich die verschiedenen Bereiche des mit der Beschichtung versehenen Festkörpers, in welchen sich unterschiedliche Korrosionsgeschwindigkeiten einstellen, hinsichtlich mindestens einer der Eigenschaften Porosität, Dicke, hydrophile/hydrophobe Eigenschaften und Zusammensetzung voneinander.

Ebenso wie die Beschichtung kann auch der Grundwerkstoff eine poröse Struktur aufweisen. Auch die Porosität dieser Struktur ist nicht notwendigerweise im gesamten Festkörper einheitlich. Beispielsweise kann eine höhere Porosität des Grundwerkstoffs in Volumenbereichen gegeben sein, die für eine vergleichsweise schnelle Biokorrosion ausgebildet sind. Dieser gewünschte Effekt kann durch eine hierauf abgestimmte Dicke und/oder Zusammensetzung der Beschichtung unterstützt werden. Insbesondere kann hierbei die Beschichtung die Biokorrosion des Grundwerkstoffs nur in einem geringen Maße behindern oder - indem sie zum Beispiel die Einstellung eines bestimmten pH-Wertes bewirkt - sogar fördern.

Bei dem biokorrodierbaren Festkörper kann es sich grundsätzlich um einen Gegenstand beliebiger, nicht notwendigerweise starrer Geometrie handeln. Weist der biokorrodierbare Festkörper beispielsweise eine langgestreckte, im Wesentlichen zylindrische Form auf, so kann dessen Dicke über die Länge des Festkörpers variieren. In vergleichbarer Weise kann auch ein Festkörper mit flächiger Gestalt eine uneinheitliche Dicke aufweisen.

Auf einen Festkörper mit einheitlicher Dicke kann entweder eine Beschichtung mit einheitlichen Biokorrosionseigenschaften oder eine Beschichtung mit von Flächenabschnitt zu Flächenabschnitt unterschiedlichen Biokorrosionseigenschaften aufgetragen werden. Die je nach Oberflächenabschnitt unterschiedlichen Eigenschaften, was die Biodegradierbarkeit der Beschichtung betrifft, können entweder durch die Beschichtungsdicke oder durch sonstige Parameter der Beschichtung gegeben sein. In jedem Fall existieren unterschiedliche Möglichkeiten der Korrelation zwischen der Biodegradierbarkeit der Beschichtung einerseits und der Biodegradierbarkeit des Grundwerkstoffs andererseits, wobei die Abbaubarkeit des Grundwerkstoffs insbesondere durch dessen Dicke, jedoch auch durch sonstige Eigenschaften, insbesondere die Materialzusammensetzung und/oder Porosität, bestimmt sein kann.

Ist eine positive Korrelation zwischen der Biodegradierbarkeit der Beschichtung und der Biodegradierbarkeit des Grundwerkstoffs gegeben, so heißt dies, dass eine schnelle Abbaubarkeit der Beschichtung in einem bestimmten Bereich des Festkörpers mit einer ebenfalls schnellen Abbaubarkeit des Grundwerkstoffs in dem entsprechenden Bereich einhergeht. Abweichend hiervon sind auch Fälle möglich, in denen gerade die Bereiche des Festkörpers mit geringster Materialstärke am spätesten abgebaut werden sollen. Dies ist dadurch erreichbar, dass die dünnsten Abschnitte des Festkörpers mit einer besonders langsam abbaubaren Beschichtung versehen werden. In diesem Fall wird von einer negativen Korrelation zwischen der Biodegradierbarkeit der Beschichtung und der insbesondere durch die Materialdicke beeinflussten Biodegradierbarkeit des Grundwerkstoffs gesprochen.

Bei dem Grundwerkstoff des biokorrodierbaren Implantats kann es sich entweder um einen metallischen oder um einen nicht-metallischen Werkstoff handeln. Als metallischer Werkstoff kommt insbesondere Magnesium in Betracht. Als mögliche nicht-metallische Werkstoffe sind beispielhaft Polymere, insbesondere ein Poly-Lactid, Polysaccharid, Polyamid, Polyester, PLGA oder PCL, zu nennen.

Der Grundwerkstoff kann materialeinheitlich oder materialuneinheitlich aufgebaut sein. In beiden Fällen kann es sich bei dem allgemein als Festkörper bezeichneten Gegenstand auch um ein faser- oder gewebeartiges Produkt handeln. Auch das Aufbringen der Beschichtung auf biologischem Material, beispielsweise Saatgut, ist möglich.

Das Implantat kann dazu vorgesehen sein, erhebliche mechanische Belastungen aufzunehmen. Dies gilt beispielsweise für Implantate in der Handchirurgie sowie in der Fußchirurgie. Die uneinheitliche Abbaugeschwindigkeiten können in solchen Fällen zum einen allgemein dazu dienen, Abbauprodukte, beispielsweise Wasserstoff, in nicht zu hohen Raten freizusetzen und zum anderen eine längerfristige mechanische Belastbarkeit in definierten Bereichen des Implantats gezielt aufrechtzuerhalten.

Allgemein ist der Festkörper, das heißt das Implantat, beschichtbar, indem auf einen Grundwerkstoff ein biozider Stoff sowie eine Silizium enthaltende Beschichtung auf verschiedenen Oberflächenabschnitten in uneinheitlicher Weise aufgebracht wird, womit die Biodegradierbarkeit in einzelnen Bereichen des Festkörpers, insbesondere Implantats, unterschiedlich eingestellt wird. Hinsichtlich des bioziden Stoffes, welcher in der Beschichtung enthalten sein kann, wird wiederum auf die bereits erwähnte EP 1 790 224 B1 hingewiesen.

Im Rahmen des Beschichtungsverfahrens wird beispielsweise zunächst auf die gesamte Oberfläche des Grundwerkstoffs der biozide Stoff in einheitlicher Weise aufgebracht. In einem weiteren Verfahrensschritt wird die Silizium enthaltende Beschichtung auf verschiedenen Oberflächenabschnitten in uneinheitlicher Weise aufgebracht.

Alternativ kann sowohl der biozide Stoff als auch die Silizium enthaltende Beschichtung auf verschiedenen Oberflächenabschnitten des Grundwerkstoffs in uneinheitlicher Weise aufgebracht werden. In beiden Fällen ist es möglich, den bioziden Stoff sowie die weitere Beschichtung derart aufzubringen, dass der biozide Stoff erst im Zuge der Korrosion der Beschichtung freigesetzt wird.

Gemäß einer möglichen Verfahrensführung wird die Beschichtung derart auf dem Grundwerkstoff aufgebracht, dass die Beschichtung wenigstens zu Beginn der Nutzung zumindest auf einer Teilfläche des Grundwerkstoffs sauerstoffundurchlässig ist. Im Laufe der Nutzung, das heißt insbesondere des Tragens des Implantats, kann sich eine zunehmende Sauerstoffdurchlässigkeit und damit ein beschleunigter Abbau des gesamten Festkörpers ergeben.

Nachfolgend werden mehrere Ausführungsbeispiele der Erfindung anhand einer Zeichnung näher erläutert. Hierin zeigen, teilweise grob schematisiert:
- Fig. 1: in der Art eines stilisierten Röntgenbildes Strukturen einer menschlichen Hand einschließlich eines Implantates,
- Fig. 2 und 3: ausschnittsweise Knochenstrukturen der Hand nach Figur 1 mit dem Implantat in verschiedenen Korrosionsstadien,
- Fig. 4: einen möglichen Aufbau eines beschichteten, biokorrodierbaren Implantats,
- Fig. 5: ein weiteres Ausführungsbeispiel eines beschichteten, biokorrodierbaren Implantats in einer schematischen Schnittdarstellung.

Die folgenden Erläuterungen beziehen sich, soweit nicht anders angegeben, auf sämtliche Ausführungsbeispiele. Einander entsprechende Teile und Parameter sind mit den gleichen Bezugszeichen gekennzeichnet.

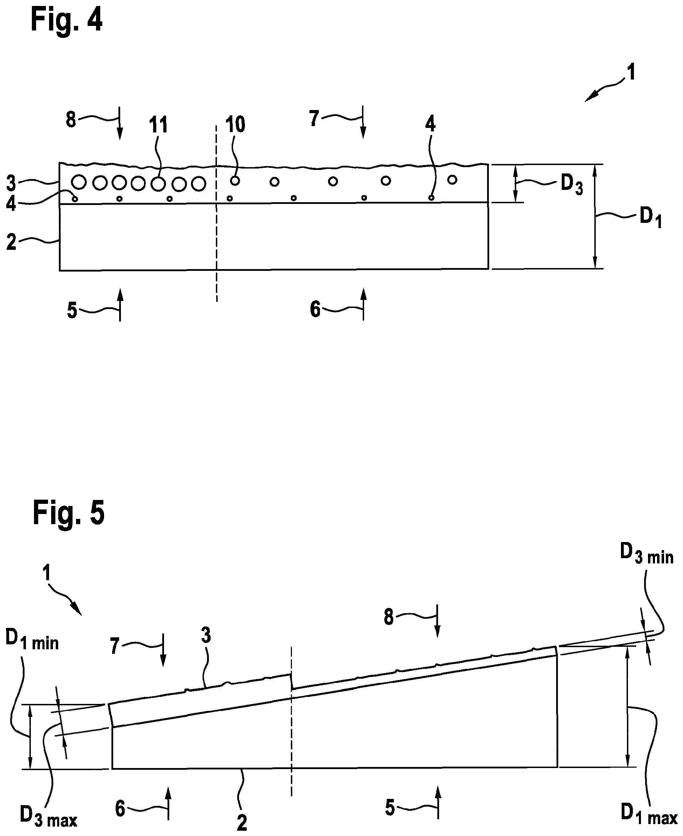

Ein medizintechnisches Implantat 1 ist als Festkörper ausgebildet, welcher aus einem Grundwerkstoff 2, in den vorliegenden Fällen einer Magnesiumlegierung, und einer hierauf befindlichen Beschichtung 3 aufgebaut ist. Die Dicke des Festkörpers 1 ist allgemein mit D₁ angegeben. Soweit der Festkörper 1, das heißt das Implantat, eine uneinheitliche Dicke aufweist, werden die Bezeichnungen D₁ₘᵢₙ für die geringste Dicke und D₁ₘₐₓ für die größte Dicke verwendet. Für die Dicke der Beschichtung 3 werden die Bezeichnungen D₃, D₃ₘᵢₙ und D₃ₘₐₓ verwendet. In den Figuren 4 und 5 ist die Beschichtung 3 übertrieben dick dargestellt. Abweichend von den symbolisierten Darstellungen kann die gesamte Oberfläche des Grundwerkstoffs 2 beschichtet sein.

Bei dem Grundwerkstoff 2 handelt es sich um ein biokorrodierbares Material. Die tatsächliche, bei im Körper des Patienten befindlichen Implantat 1 auftretende Biokorrosion wird maßgeblich durch die Dicke und Beschaffenheit der Beschichtung 3 beeinflusst. Die Beschichtung 3 enthält in den vorliegenden Fällen Silizium als Hauptbestandteil und darüber hinaus einen bioziden Stoff 4, welcher sich direkt auf der Oberfläche des Grundwerkstoffs 2 befindet. Der biozide Stoff 4 ist den skizzierten Fällen in gleichmäßiger Verteilung auf die Oberfläche des Grundwerkstoffs 2 aufgetragen.

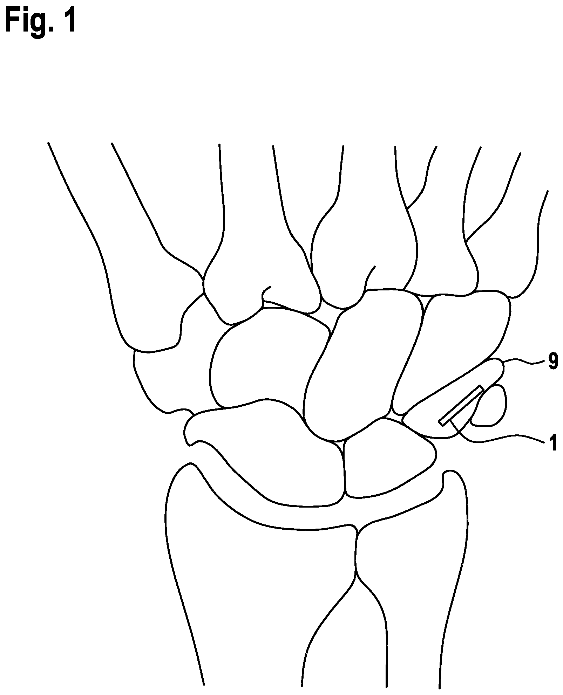

Der biokorrodierbare Festkörper 1 umfasst verschiedene Bereiche 5, 6, die sich hinsichtlich ihres Korrosionsverhaltens voneinander unterscheiden. Das unterschiedliche Korrosionsverhalten der verschiedenen Bereiche 5, 6 wird maßgeblich durch die unterschiedliche Beschaffenheit der Beschichtung 3 in den verschiedenen Bereichen 5, 6 bestimmt. Hierbei tritt in einen Beschichtungsabschnitt 7 eine langsamere, im Beschichtungsabschnitt 8 dagegen eine schnellere Biokorrosion auf. Der unterschiedlich schnelle Korrosionsangriff in den verschiedenen Bereichen 5, 6 ist in den Figuren 1 bis 3 veranschaulicht. Diese Figuren zeigen einen in einen Knochen 9 eingesetzten metallischen Festkörper 1, bei welchem es sich um ein Implantat auf Magnesiumbasis handelt.

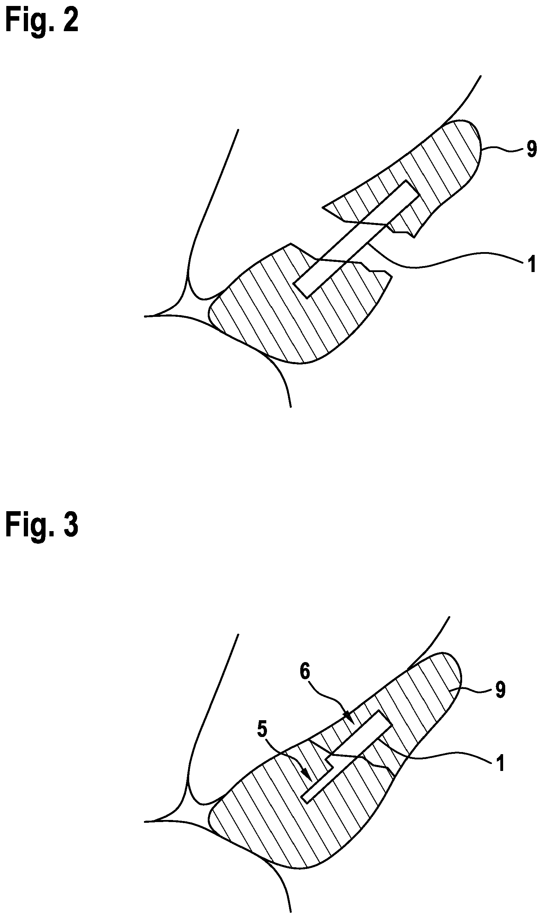

In Figur 2 ist ein Zustand beim Implantieren des Festkörpers 1, in Figur 3 der Zustand nach teilweisem Abbau des Festkörpers 1 veranschaulicht. Wie aus einem Vergleich der Figuren 2 und 3 hervorgeht, hat die Materialstärke des Festkörpers 1 in dem in Figur 3 visualisierten Zustand, einige Wochen nach der Operation (Figur 2) bereits deutlich abgenommen. Im Bereich 6 fand dagegen bis zu diesem Zeitpunkt kaum Biokorrosion statt. Mit dieser Splittung der Korrosionsgeschwindigkeiten sind zwei gewünschte Effekte verbunden: Zum einen wird bei der Biokorrosion entstehender Stoff, insbesondere Wasserstoff, über einen längeren Zeitraum mit niemals zu hoher Rate freigesetzt. Zum anderen bleibt die Verankerung des Bereichs 6 im Knochen 9 über einen längeren Zeitraum erhalten. Nach vollständiger Heilung ist das gesamte Implantat 1 durch Knochenmaterial ersetzt.

Die Figur 4 illustriert die Beschaffenheit der verschiedenen Abschnitte 7, 8 der Beschichtung 3 des im Beispiel nach den Figuren 1 bis 3 verwendeten Implantats 1. Sowohl im Abschnitt 7 als auch im Abschnitt 8 der Beschichtung 3 ist eine Porosität erkennbar, wobei Poren mit 10, 11 bezeichnet sind und in unterschiedlicher Größe und Verteilung in den verschiedenen Abschnitten 7, 8 vorhanden sind. Mit bloßem Auge ist die unterschiedliche Beschaffenheit der Beschichtung 3 in den verschiedenen Abschnitten 7, 8 in der Regel nicht erkennbar. Die unterschiedliche Verteilung und mittlere Größer der Poren 10, 11 sorgt jedoch für ein signifikant unterschiedliches Biokorrosionsverhalten der verschiedenen Abschnitte 7, 8 in der gegebenen biologischen Umgebung. Dies ist insbesondere auf die unterschiedlichen hydrophilen beziehungsweise hydrophoben Eigenschaften der Beschichtungsabschnitte 7, 8 zurückzuführen. Im Beschichtungsabschnitt 7, der sich durch einen geringen Korrosionsangriff auszeichnet, sind hydrophobe Eigenschaften der Beschichtung 3 gegeben, welche für einen längerfristigen Schutz des Grundwerkstoffs 2 sorgen, wobei kaum eine Sauerstoffdurchlässigkeit der Beschichtung 3 im Abschnitt 7 gegeben ist. Dagegen sind in dem eine höhere Porosität aufweisenden Abschnitt 8 der Beschichtung 3 hydrophile Eigenschaften eingestellt, welche mit einer rascheren Biokorrosion einhergehen.

Die Figur 5 zeigt eine alternative Möglichkeit, unterschiedliche Biokorrosionseigenschaften in den verschiedenen Bereichen 5, 6 des Festkörpers 1 einzustellen. Auch diese Variante ist für den Anwendungsfall nach den Figuren 1 bis 3 geeignet. Die verschiedenen Abschnitte 7, 8 der Beschichtung 3 weisen in diesem Fall eine einheitliche Zusammensetzung auf und unterscheiden sich lediglich hinsichtlich ihrer Dicke D₃ₘᵢₙ, D₃ₘₐₓ voneinander. Im Unterschied zu dem in Figur 4 skizzierten Beispiel weist der Grundwerkstoff 2 und damit der gesamte Festkörper 1 im Fall von Figur 5 eine uneinheitliche Materialstärke auf. Hierbei ist eine negative Korrelation zwischen der Dicke der Beschichtung 3 und der Dicke des Grundwerkstoffs 2 gegeben. Dies bedeutet, dass die Beschichtung 3 dort am dünnsten ist, wo der Grundwerkstoff 2 am dicksten ist. Die Korrosion des Grundwerkstoffs 2 beginnt somit in seinem dicksten Bereich 5. Im vergleichsweise dünnen Bereich 6 des Implantats 1 tritt dagegen die Biokorrosion mit stärkerer Verzögerung ein. Letztlich sorgt dies dafür, dass die verschiedenen Bereiche 5, 6 etwa zur gleichen Zeit vollständig abgebaut sind. Abweichend von der vereinfachten Darstellung nach Figur 5 können auch mehr als zwei verschiedene Abschnitte 7, 8 der Beschichtung 3 ausgebildet oder kontinuierliche Übergänge zwischen verschiedenen Beschichtungsabschnitten 7, 8 gegeben sein. Dies gilt in analoger Weise auch für die Materialbeschaffenheit der verschiedenen Beschichtungsabschnitte 7, 8 des Implantats 1 nach Figur 4.

### Bezugszeichenliste

- 1: Festkörper, Implantat
- 2: Grundwerkstoff
- 3: Beschichtung
- 4: biozider Stoff
- 5: Bereich des Festkörpers
- 6: Bereich des Festkörpers
- 7: Oberflächenabschnitt mit langsamerer Biokorrosion
- 8: Oberflächenabschnitt mit schnellerer Biokorrosion
- 9: Knochen
- 10: Pore
- 11: Pore

- D₁, D₃, D₁ₘᵢₙ, D₁ₘₐₓ, D₃ₘᵢₙ, D₃ₘₐₓ: Dicke

## Patentansprüche

1. Biokorrodierbarer Festkörper (1), nämlich Implantat, mit einem Grundwerkstoff (2), welcher in seinem Volumen eine uneinheitliche Porosität aufweist, und einer Beschichtung (3), wobei die Beschichtung (3) Silizium umfasst und derart auf den Grundwerkstoff (2) aufgebracht ist, dass sich unterschiedliche Korrosionsgeschwindigkeiten in verschiedenen Bereichen (7, 8) des Festkörpers (1) einstellen, wobei sich die verschiedenen Bereiche (7, 8) hinsichtlich mindestens einer der Eigenschaften Porosität, Dicke (D₃ₘᵢₙ, D₃ₘₐₓ), hydrophile/ hydrophobe Eigenschaften und Zusammensetzung voneinander unterscheiden und die Biodegradierbarkeit der Beschichtung (3) mit der Dicke (D₁, D₁ₘᵢₙ, D₁ₘₐₓ) des Grundwerkstoffs (2) negativ korreliert ist.

2. Festkörper (1) nach Anspruch 1, **gekennzeichnet durch** einen zwischen dem Grundwerkstoff (2) und der Beschichtung (3) befindlichen bioziden Stoff (4).

3. Festkörper (1) nach Anspruch 1 oder 2, **dadurch gekennzeichnet, dass** die Beschichtung (3) ebenso wie der Grundwerkstoff (2) eine poröse Struktur aufweist.

4. Festkörper (1) nach einem der Ansprüche 1 bis 3, **dadurch gekennzeichnet, dass** der Grundwerkstoff (2) Magnesium als Hauptbestandteil enthält.

5. Festkörper (1) nach einem der Ansprüche 1 bis 3, **dadurch gekennzeichnet, dass** der Grundwerkstoff (2) ein Polymer, insbesondere ein Poly-Lactid, Polysaccharid, Polyamid, Polyester, PLGA oder PCL, umfasst.

6. Festkörper (1) nach einem der Ansprüche 1 bis 4, **dadurch gekennzeichnet, dass** der Grundwerkstoff (2) im Gegensatz zur Beschichtung einheitlich aufgebaut ist.

7. Festkörper (1) nach einem der Ansprüche 1 bis 5, **dadurch gekennzeichnet, dass** dieser faser- oder gewebeartig ausgebildet ist.

8. Festkörper (1) nach Anspruch 1, **dadurch gekennzeichnet, dass** es sich bei diesem um biologisches Material handelt.

9. Festkörper (1) nach Anspruch 1, zur Verwendung in der Handchirurgie oder Fußchirurgie.

10. Verfahren zur Beschichtung eines biokorrodierbaren Festkörpers (1), nämlich Implantats, insbesondere Magnesium-Implantats, wobei auf einen Grundwerkstoff (2), welcher in seinem Volumen eine uneinheitliche Porosität aufweist, ein biozider Stoff (4) sowie eine Silizium enthaltende Beschichtung (3) auf verschiedenen Oberflächenabschnitten (7, 8) in uneinheitlicher Weise derart aufgebracht wird, dass sich verschiedene Bereiche (5, 6) hinsichtlich mindestens einer der Eigenschaften Porosität, Dicke (D₃ₘᵢₙ, D₃ₘₐₓ), hydrophile/ hydrophobe Eigenschaften und Zusammensetzung voneinander unterscheiden, womit die Biodegradierbarkeit in den einzelnen Bereichen (5, 6) des Implantats, mit einer negativen Korrelation zwischen der Biodegradierbarkeit der Beschichtung (3) und der Dicke (D₁, D₁ₘᵢₙ, D₁ₘₐₓ) des Grundwerkstoffs (2), unterschiedlich eingestellt wird.

11. Verfahren nach Anspruch 10, **dadurch gekennzeichnet, dass** zunächst auf die gesamte Oberfläche des Grundwerkstoffs (2) der biozide Stoff (4) in einheitlicher Weise aufgebracht wird und in einem weiteren Verfahrensschritt die Silizium enthaltende Beschichtung (3) auf verschiedenen Oberflächenabschnitten (7, 8) in uneinheitlicher Weise aufgebracht wird.

12. Verfahren nach Anspruch 10, **dadurch gekennzeichnet, dass** sowohl der biozide Stoff (4) als auch die Silizium enthaltende Beschichtung (3) auf verschiedenen Oberflächenabschnitten (7, 8) des Grundwerkstoffs (2) in uneinheitlicher Weise aufgebracht wird.

13. Verfahren nach einem der Ansprüche 10 bis 12, **dadurch gekennzeichnet, dass** der biozide Stoff (4) sowie die weitere Beschichtung (3) derart aufgebracht werden, dass der biozide Stoff (4) erst im Zuge der Korrosion der Beschichtung (3) freigesetzt wird.

14. Verfahren nach einem der Ansprüche 10 bis 13, **dadurch gekennzeichnet, dass** der Aufbau der Beschichtung (3) derart eingestellt wird, dass diese zumindest zu Beginn der Nutzung sauerstoffundurchlässig ist.

## Claims

1. A biocorrodible solid body (1), specifically an implant, with a base material (2), which has a non-uniform porosity in its volume, and with a coating (3), wherein the coating (3) comprises silicon and is applied to the base material (2) such that different corrosion rates are established in different areas (7, 8) of the solid body (1), wherein the different areas (7, 8) differ from one another with regard to at least one of the properties of porosity, thickness (D₃ₘᵢₙ, D₃ₘₐₓ), hydrophilic/hydrophobic properties and composition, and the biodegradability of the coating (3) is negatively correlated with the thickness (D₁, D₁ₘᵢₙ, D₁ₘₐₓ) of the base material (2).

2. The solid body (1) according to claim 1, **characterised by** a biocidal substance (4) located between the base material (2) and the coating (3).

3. The solid body (1) according to claim 1 or 2, **characterised in that** the coating (3) and also the base material (2) have a porous structure.

4. The solid body (1) according to any one of claims 1 to 3, **characterised in that** the base material (2) contains magnesium as main constituent.

5. The solid body (1) according to any one of claims 1 to 3, **characterised in that** the base material (2) comprises a polymer, in particular a polylactide, polysaccharide, polyamide, polyester, PLGA or PCL.

6. The solid body (1) according to any one of claims 1 to 4, **characterised in that** the base material (2), in contrast to the coating, is uniformly structured.

7. The solid body (1) according to any one of claims 1 to 5, **characterised in that** it is fibre-like or fabric-like.

8. The solid body (1) according to claim 1, **characterised in that** it is biological material.

9. The solid body (1) according to claim 1 for use in hand surgery or foot surgery.

10. A method for coating a biocorrodible solid body (1), specifically an implant, in particular magnesium implant, wherein a biocidal substance (4) and also a silicon-containing coating (3) are applied in a non-uniform manner to a base material (2), which in its volume has a non-uniform porosity, on different surface portions (7, 8) in such a way that different areas (5, 6) differ from one another with regard to at least one of the properties of porosity, thickness (D₃ₘᵢₙ, D₃ₘₐₓ), hydrophilic/hydrophobic properties and composition, whereby the biodegradability in the individual areas (5, 6) of the implant is set differently, with a negative correlation between the biodegradability of the coating (3) and the thickness (D₁, D₁ₘᵢₙ, D₁ₘₐₓ) of the base material (2).

11. The method according to claim 10, **characterised in that** the biocidal substance (4) is first applied in a uniform manner to the entire surface of the base material (2) and in a further method step the silicon-containing coating (3) is applied in a non-uniform manner to different surface portions (7, 8).

12. The method according to claim 10, **characterised in that** both the biocidal substance (4) and the silicon-containing coating (3) are applied in a non-uniform manner to different surface portions (7, 8) of the base material (2).

13. The method according to any one of claims 10 to 12, **characterised in that** the biocidal substance (4) and the further coating (3) are applied in such a way that the biocidal substance (4) is only released during the course of the corrosion of the coating (3).

14. The method according to any one of claims 10 to 13, **characterised in that** the structure of the coating (3) is set in such a way that it is impermeable to oxygen at least at the start of the use.

## Revendications

1. Corps solide biocorrodable (1), à savoir un implant, doté d'une matière de base (2), qui présente dans son volume une porosité non homogène et d'un revêtement (3), le revêtement (3) comprenant du silicium et étant appliqué sur la matière de base (2) de telle sorte que différentes vitesses de corrosion s'établissent dans différentes zones (7, 8) du corps solide (1), les différentes zones (7, 8) se distinguant les unes des autres en ce qui concerne au moins l'une des propriété que sont la porosité, l'épaisseur (D₃ₘᵢₙ, D₃ₘₐₓ), les propriétés hydrophiles / hydrophobes et la composition et la biodégradabilité du revêtement (3) étant négativement corrélée avec l'épaisseur (D₁, D₁ₘᵢₙ, D₁ₘₐₓ) de la matière de base (2).

2. Corps solide (1) selon la revendication 1, **caractérisé par** une substance biocide (4) située entre la matière de base (2) et le revêtement (3).

3. Corps solide (1) selon la revendication 1 ou 2, **caractérisé en ce que** le revêtement (3) comporte tout aussi bien que la matière de base (2) une structure poreuse.

4. Corps solide (1) selon l'une quelconque des revendications 1 à 3, **caractérisé en ce que** la matière de base (2) comporte en tant que composant principal du magnésium.

5. Corps solide (1) selon l'une quelconque des revendications 1 à 3, **caractérisé en ce que** la matière de base (2) comprend un polymère, notamment un polylactide, un polysaccharide, un polyamide, un polyester, du PLGA ou de la PCL.

6. Corps solide (1) selon l'une quelconque des revendications 1 à 4, **caractérisé en ce que** contrairement au revêtement, la matière de base (2) est constituée de manière homogène.

7. Corps solide (1) selon l'une quelconque des revendications 1 à 5, **caractérisé en ce que** ce dernier est conçu de type fibreux ou de type textile.

8. Corps solide (1) selon la revendication 1, **caractérisé en ce qu'**il s'agit d'un matériau biologique.

9. Corps solide (1) selon la revendication 1, destiné à être utilisé dans la chirurgie de la main ou la chirurgie du pied.

10. Procédé, destiné à revêtir un corps solide (1) biocorrodable, à savoir un implant, notamment un implant en magnésium, lors duquel, sur une matière de base (2), laquelle présente dans son volume une porosité non homogène, l'on applique de manière non homogène sur différentes parties superficielles (7, 8) une substance biocide (4), ainsi qu'un revêtement (3) contenant du silicium de telle sorte que différentes zones (5, 6) se distinguent les unes des autres en ce qui concerne au moins l'une des propriétés que sont la porosité, l'épaisseur (D₃ₘᵢₙ, D₃ₘₐₓ), les propriétés hydrophiles / hydrophobes et la composition, suite à quoi, la biodégradabilité dans les zones (5, 6) individuelles de l'implant s'établit de manière différente, avec une corrélation négative entre la biodégradabilité du revêtement (3) et l'épaisseur (D₁, D₁ₘᵢₙ, D₁ₘₐₓ) de la matière de base (2).

11. Procédé selon la revendication 10, **caractérisé en ce que** l'on applique d'abord de manière homogène la substance biocide (4) sur l'ensemble de la surface de la matière de base (2) et lors d'une étape de procédé supplémentaire, l'on applique de manière non homogène le revêtement (3) contenant du silicium sur différentes parties superficielles (7, 8).

12. Procédé selon la revendication 10, **caractérisé en ce que** l'on applique de manière non homogène aussi bien la substance biocide (4) qu'également le revêtement (3) contenant du silicium sur différentes parties superficielles (7, 8) de la matière de base (2).

13. Procédé selon l'une quelconque des revendications 10 à 12, **caractérisé en ce que** l'on applique la substance biocide (4) ainsi que le revêtement (3) supplémentaire de telle sorte que la substance biocide (4) ne soit libérée qu'au fur et à mesure de la corrosion du revêtement (3).

14. Procédé selon l'une quelconque des revendications 10 à 13, **caractérisé en ce que** l'on établit la constitution du revêtement (3) de telle sorte qu'au moins au début de l'utilisation, celui-ci soit imperméable à l'oxygène.
